**Europäisches Patentamt**

(19) **European Patent Office**

**Office européen des brevets**

(11) Publication number : **0 214 271**
**B1**

(12) # EUROPEAN PATENT SPECIFICATION

(45) Date of publication of patent specification :
01.02.89

(51) Int. Cl.⁴ : **C 12 M   1/00, A 01 G 33/00**

(21) Application number : **86902058.6**

(22) Date of filing : **06.03.86**

(86) International application number :
**PCT/SE 86/00094**

(87) International publication number :
**WO/86052 (12.09.86 Gazettee 86/20)**

(54) **PLANT FOR CULTIVATING ALGAE.**

(30) Priority : **06.03.85 SE 8501092**

(43) Date of publication of application :
**18.03.87 Bulletin 87/12**

(45) Publication of the grant of the patent :
**01.02.89 Bulletin 89/05**

(84) Designated contracting states :
**BE CH DE FR GB IT LI NL SE**

(56) References cited :
**GB—A— 1 451 699**
**GB—A— 2 118 572**
**US—A— 2 715 795**
**US—A— 4 473 970**
**Derwent's Abstract no. 84-206402**
**Derwent's Abstract no. 81-67396**
**Derwent's Abstract no. 79-36007**
**J.Chem.Tech.Biotechnol., vol. 33B, pp. 35-58, publ.**
**1983, S. Pirt et al. "A tubular bioreactor to photo-**
**synthetic production of biomass from carbon dioxide:**
**Design and Performance"**

(73) Proprietor : **RYGAARD, Sven**
**Lojovägen 57**
**S-181 47 Lidingo (SE)**

(72) Inventor : **RYGAARD, Sven**
**Lojovägen 57**
**S-181 47 Lidingo (SE)**

(74) Representative : **Nordén, Ake et al**
**AWAPATENT AB Box 7402**
**S-103 91 Stockholm (SE)**

## Description

The present invention relates to a plant for cultivating algae, preferably microalgae, comprising a tank in which the algae are allowed to grow in circulating water and which is provided with walls having light openings through which the water with the growing algae is illuminated.

It has long been known that certain types of green algae growing substantially in fresh and brackish water can be used as high-protein foods both for human beings and animals. Today, scientific research is conducted in many places throughout the world in order to determine and catalogue the different forms of existing marine and fresh water algae and also to determine the usefulness of the various types and their value as a basis for industrial processes and systems with a view to establishing, analyzing and verifying their valuable contents and their usefulness as a basis for industrial processes and systems. It is essential to create an adequate and pure environment without any irrelevant organisms and otherwise optimum conditions in order to maintain and promote the growth rate of the algae in a cost-effective way for developing and recovering their valuable contents for the food, chemical, medical/pharmaceutical industries as well as modern agriculture. On a long view, it may also become possible to recover e. g. hydrogen gas and other substances of interest as by-products in such processes.

All this is at present well known in the art. It is also known from innumerable laboratory experiments and small-scale test cultivations which are the most favourable conditions and what environmental factors should be considered and which of these factors should be avoided. Also, it is known that it is uneconomical to conduct cultivation depending on the sunlight. Here, as in other instances, the sun has proved to be too an expensive and unreliable source of energy although the algae can use the sunlight better than most other useful plants.

Previous experiments have shown that, contrary to expectations, continuous exposure to daylight or strong illumination with different lamps for extended periods with high power and continuity is not the ideal solution to the problem. A relatively weak artificial illumination alternating with relatively long dark periods is more favourable.

The object of the invention is to provide a plant in which a highly diluted dispersion of microalgae can be caused to grow. The plant should also be so arranged as to allow continuous production of algae in a rapid and efficient manner and, moreover, a high degree of automation. Further, the plant should permit complete control in respect of the quality of the algae produced and prevent the appearance of contaminations in the form of foreign organisms.

The essential features of the plant according to the invention reside in that the tank in which the water admixed with nutrient solution is caused to circulate consists of a closed channel system or the like being designed as one or more helical tube coils suitably arranged in pairwise communicating piles, that the light-transmitting portions are suitably arranged in the tube sides along substantially axial regions at said coils or piles, and that one or more light sources, preferably in the form of fluorescent lamps, are provided at the light-transmitting portions.

By means of the plant according to the invention, large amounts of algae, e. g. for human and animal food or as raw material for the chemical industry, can be produced under carefully controllable hygienic conditions and at a cost making it possible to use the algal mass in many fields.

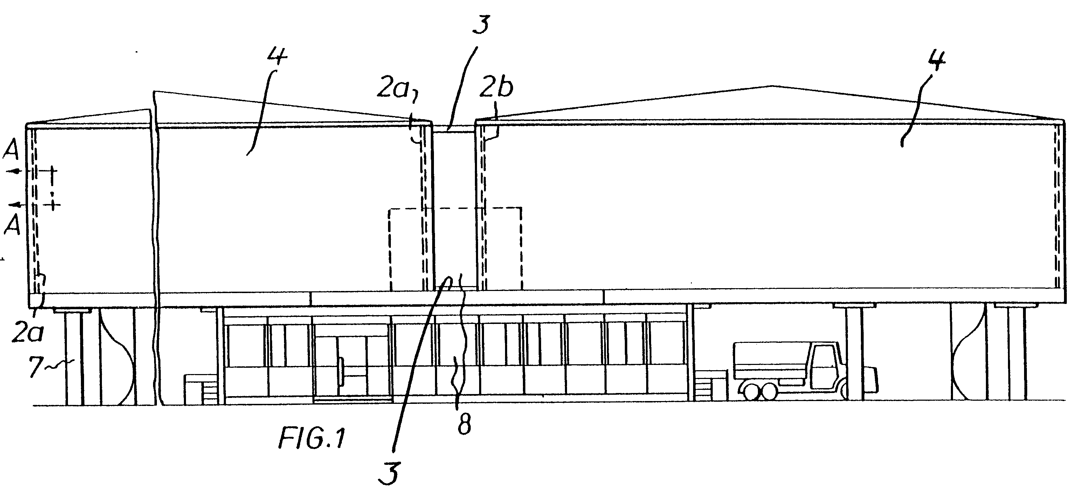

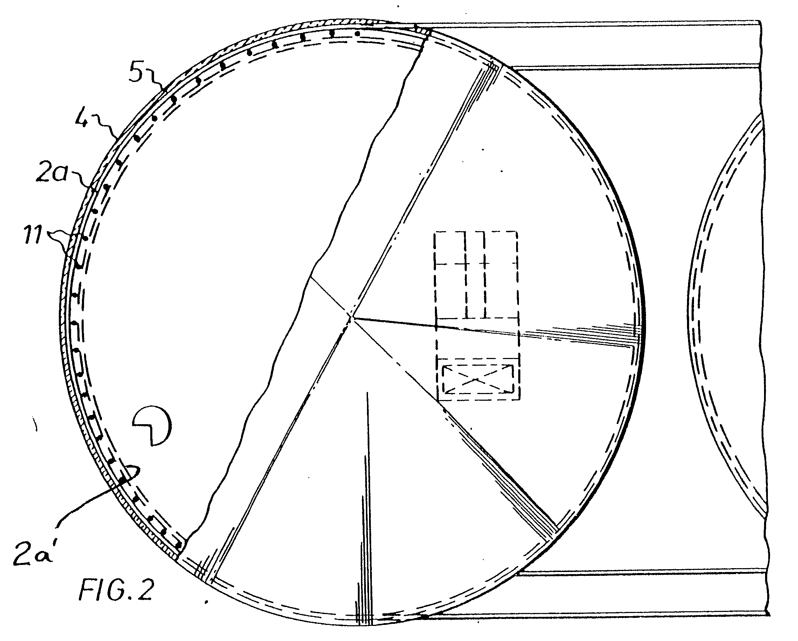

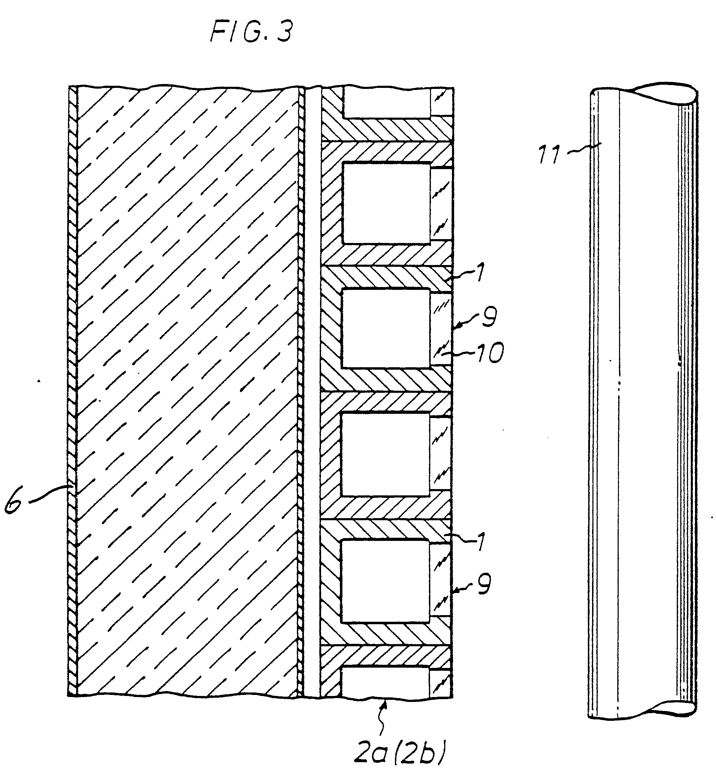

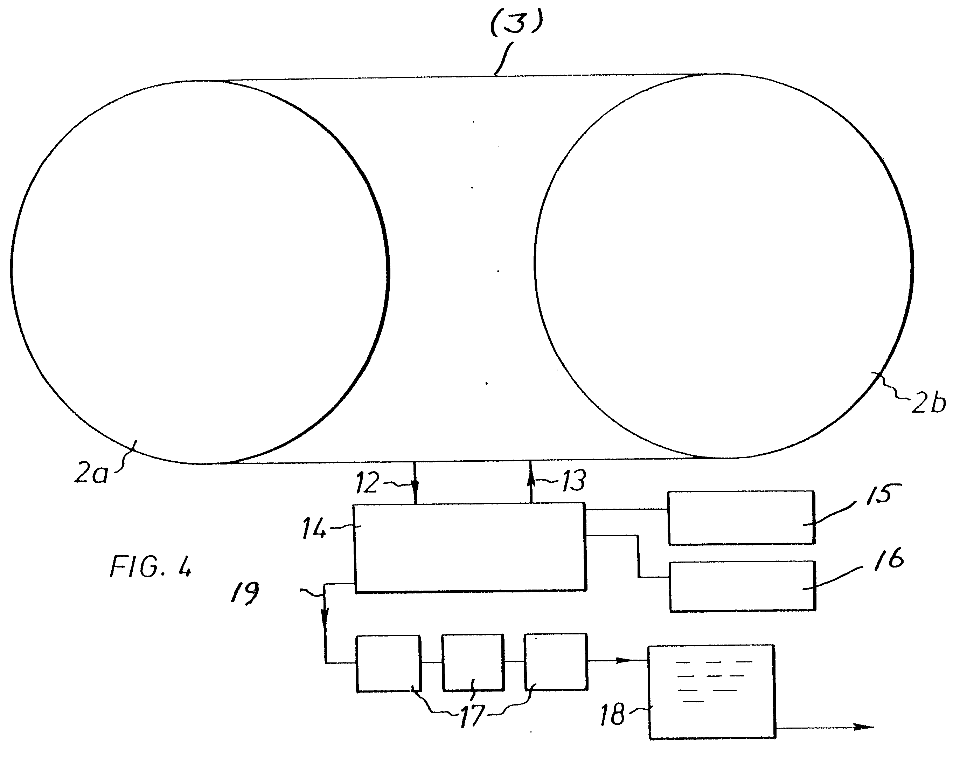

The invention will be described in more detail hereinbelow with reference to the accompanying drawings in which Fig. 1 shows the exterior of a plant of the type concerned, Fig. 2 is a horizontal crosssection of one half of the plant, and Fig. 3 is a section on a larger scale taken along the line A-A in Fig. 1, and Fig. 4 schematically illustrates the design of a preferred embodiment.

In the preferred embodiment, the main part of the plant consists of a helically extending tube coil 1 arranged in two interconnected piles 2a and 2b, the coils being so connected that liquid can flow upwards in one pile and downwards in the other. Between the tube coils, there are connected pump means indicated at 3 as well as further assemblies to be described in more detail hereinbelow.

The tube coils or piles 2a, 2b form the « inner wall » in the tower-like structures 4 shown in Fig. 1, and outwardly of the tube piles there are provided an insulation 5 and an outer covering 6. The tower-like structures which are provided with suitable temperaturemaintaining means and the interior of which can be used for any suitable purpose are of course provided with an insulated roof and rest on pillars 7. Pumps 3 and other necessary equipment are provided in spaces 8 defined between and partly underneath the structures containing the tube piles.

The coils in the tube piles are provided at mutual intervals with openings 9 which are covered by glass or other material and are indicated in Fig. 3.

In the preferred embodiment, the tubes 1 forming the piles are substantially square in crosssection, which makes it possible, without any major problems, to provide an opening 9 in their inwardly facing side and cover it with glass 10.

Inwardly of the respective tube pile 2a, 2b adjacent each row of openings 9, there are disposed a number of luminescent lamps or like light sources 11, such that light from the fluorescent lamps can be flashed in through the openings in the tube coils.

As previously mentioned, the tube piles 2a, 2b communicating with each other are connected to pump means 3 for circulating the liquid as required. Further, there are provided dosage means (not shown) for supplying nutrient solution, a parent solution, i. e. an aqueous dispersion, and algal substance to be grown, and for supplying carbon dioxide, suitably mixed with air. There are of course also discharge means leading to preliminary dewatering centrifuging means and from there conduits for recycling liquid spun off containing non-spent nutrient solution.

In the lower portions of the tower-like structures ; there may be provided apparatus for treating the partly dewatered algal mass which is adequately recovered, packaging means and the like.

As previously indicated, it is important for the algal growth that light is intermittently supplied. As is known, continuous illumination does not give the desired growth whereas intermittent illumination during relatively short periods has been found to favourably affect the growth. The fluorescent lamps 11 in combination with the light openings 9 in the tubes make the liquid, when passing such a light opening, momentarily illuminated by a relatively intense light.

In order that the intended growth should take place, use is made of a nutrient solution in which algal growth occurs. As mentioned above, the highly viscous mass of algae and nutrient solution is also supplied with carbon dioxide in a relatively large amount. Essential for algal growth is also that the mass is subjected to turbulence, such that the nutrient solution comes into intimate contact with the growing algae in order to optimize nutrient absorption.

Since carbon dioxide (suitably mixed with air) is supplied under pressure, turbulence is increased not only at the moment of supply but also during the circulation of the liquid/algal mass through the tube system, which results in that each separate portion of the suspension will sooner or later be located adjacent the light openings and be illuminated. The supply of carbon dioxide and air can be effected in conjunction with the pump as indicated at 3. The liquid on the delivery side of the pump has in fact greater capacity of maintaining gas in solution than the liquid on the suction side of the pump. It should be noted that the carbon dioxide content should be kept at a relatively high level of about 3-5 %.

The cross-sectional area of the tubes is also of importance for the yield. The smaller the tube area is, the larger becomes the yield. As is well known, a smaller tube gives improved turbulence and, in small tubes, there is obtained a notable improvement of the effect of light. In tubes having a small cross-sectional area and provided with « windows », the light can penetrate deeper into and affect the entire fluid body passing the window.

It may here be added that the water spun off in the final stage, if the content of nutrient solution is considered small and it is not recycled into the circulation system, can be used for fish-breeding plants. In fact, the water contains, also after centrifugation, a certain amount of algae of such a size that they cannot be used for their primary purpose. However, they are very well suited as fish fodder.

The withdrawal of fully developed algal mass can be effected once a day or at intervals depending on the growth rate. The addition of parent solution is effected at similar intervals. The dosage of nutrient solution, like the addition of carbon dioxide, is suitably controlled automatically during the entire growth period.

The concrete design of the plant as regards e. g. the pitch of the coils is determined by the desired flow velocity. After the process has been started and since the channel system is not completely emptied, it is necessary, after the initial filling, to supply but a relatively limited amount of parent solution, after each emptying operation. The remaining dispersion left in the channel system in fact serves per se as parent solution.

Modifications of the design are of course possible. Thus, a second tube pile 2a', indicated by broken lines in Fig. 2, may for instance be disposed inside each of the tube piles 2a and 2b, and the tubes in this pile may be provided with external glazed openings which are illuminated by the same luminescent lamps as the openings 9 in the outer tube piles. It may thus be possible to almost double the production capacity with a substantially unaltered consumption of light energy. Since, in such a case, the luminescent lamps would become more difficult of access, it would be necessary to make the luminescent lamp fittings retractable, for instance downwardly for maintenance and exchange.

In the embodiment illustrated in Fig. 4, there are two tube coils 2a and 2b only schematically indicated which may be doubled.

The tube coils are interconnected in the manner previously described and in the connection between them, there are two branchlines 12 and 13 conducting to an expansion and settling tank 14 to which are connected tanks 15 and 16 for $CO_2$ and air/nitrogen, respectively.

From the lower portion of the expansion and settling tank, a conduit 19 leads to dewatering devices 17 which, in the illustrated embodiment, consist of one or more centrifuges or the like. The last centrifuge communicates with a final treatment device 18, suitably comprising a freezing plant.

It has been found suitable to supplement white fluorescent lamps with red and blue ones since such a light combination gives the best yield.

To adjust the period of time during which the algal solution is exposed to light, the length of the openings 9 in the tube piles 2a, 2b can be adjusted in relation to the flow velocity. In the illustrated example, openings have been chosen having a length of about 150 mm and spaced from each other about 3 m in the longitudinal direction of the tube.

In one embodiment, there are 100 light-emitting stations arranged adjacent a tube pile containing 30 superposed tubes. Adjacent each light-emitting station, the tubes are provided with light openings, i. e. 30 light openings in succession above each other.

In a mechanical light control system for such a plant, each light-emitting station comprises a motorpowered rotary cylinder surrounding the light sources, suitably consisting of fluorescent lamps, and provided with longitudinal slots which during rotation give rise to a light flash sweeping over the light openings in the tubes and alternating with dark periods. The suspension circulating through the tubes will thus be illuminated by light flashes of short duration. The ratio of light to dark may be approximately 1 :10, and the light flashes normally have very short duration, down to milliseconds.

At the light-emitting stations, the duration of the light flashes can be regulated by adjusting the slot width and the speed of rotation in relation to the flow velocity of the suspension past the light openings. The cylinders may be either co- or counterrotating in relation to the suspension.

A further possibility of controlling and adjusting the light flashes is had if the light sources are caused to emit intermittent light electronically.

As mentioned above, the essential thing is that each individual cell suspended in the suspension is subjected to a light flash and, in this context, the turbulence in the tubes is of importance.

## Claims

1. A plant for cultivating algae, preferably microalgae, comprising a tank in which the algae are allowed to grow in circulating water and which consists of a closed channel system or the like, being designed as one or more helical tube coils suitably arranged in pairwise communicating piles (2a, 2b) and provided with light-transmitting portions (9), and one or more light sources, preferably in the form of fluorescent lamps (11) provided at the lighttransmitting portions (9), characterized in that at least two helical tube coils or piles (2a, 2b) in which the tubes (1) provided with light-transmitting portions and forming the coils or piles and preferably having rectangular cross-section are disposed close to each other, form part of the channel system provided in a temperate space and also comprising means for supplying an algal dispersion serving as parent solution, nutrient solution, $CO_2$, air and the like, and sufficient expansion space and means for withdrawing water with fully developed algae, that the tube coils or piles (2a, 2b) are so interconnected that in a pair of such coils or piles the flow occurs upwardly in one and downwardly in the other of said coils or piles, and that the light-transmitting portions (9) consist of scattered openings in the otherwise light-impervious tube wall, are covered by plates or portions (10) of glass or like translucent material and seen in the longitudinal direction of the tube have a significantly smaller length than the intermediate light-impervious portions.

2. Plant as claimed in claim 1, characterized in that mechanical screen means in the form of rotary cylinders provided with slots or like openings but otherwise impervious to light are arranged around the light sources in order to adjust the length of time the light sources project light towards and through the light-transmitting portions (9) of the tubes in the tube piles.

3. Plant as claimed in claim 1, characterized in that electronic control means are connected to the light sources and adapted to cause these to emit light during short periods interrupted by dark periods.

4. Plant as claimed in claim 1, characterized in that the light penetrating through at least some of the light-transmitting portions and into the circulating algal dispersion has different colours, suitably white, red and blue.

5. Plant as claimed in claim 1, characterized in that the ratio of the time during which the algae in the liquid are kept in dark to the time during which they are illuminated is 10 : 1.

## Patentansprüche

1. Anlage zur Zucht von Algen, vorzugsweise Mikroalgen, mit einem Gefäss, in welchem die Algen in strömendem Wasser wachsen, welches aus einem geschlossenen, mit geringer Fläche und grosser Länge als eine oder mehrere schraubenlinienförmige Rohrschleifen ausgeführten Kanalsystem oder dergleichen besteht, wobei die Rohrschleifen zweckmässigerweise in paarweise miteinander in Verbindung stehenden Stapeln (2a, 2b) angebracht und mit lichtdurchlässigen Abschnitten (9) sowie einer oder mehreren Lichtquellen, vorzugsweise in Form von an den lichtdurchlässigen Abschnitten (9) angebrachten Leuchtröhren (11) versehen sind, dadurch gekennzeichnet, dass wenigstens zwei schraubenlinienförmig angeordnete Rohrschleifen oder -stapel (2a, 2b), in denen die mit lichtdurchlässigen Abschnitten versehenen, die Schleifen oder Stapelbildenden Rohre (1) vorzugsweise rechteckigen Querschnitt haben, dicht aneinander angrenzen und zu dem in einem temperierten Raum vorgesehenen Kanalsystem gehören, welches auch Mittel zur Zuführung von als Stammlösung dienender Algendispersion, Nahrungslösung, $CO_2$, Luft und der gleichen sowie erforderlichen Ausdehnungsraum und Vorrichtungen zum Ablassen von Wasser mit fertig entwickelten Algen umfasst, dass die Rohrschleifen oder -stapel (2a, 2b) so miteinander verbunden sind, dass in einem Paar solcher Stapel bzw. Schleifen die Strömung einesteils nach oben und anderenteils nach unten verläuft und dass die lichtdurchlässigen Abschnitte (9) aus verteilten Öffnungen in der sonst lichtdichten Rohrwand bestehen, die durch Platten oder Abschnitte (10) aus Glas oder ähnlichem durchsichtigem Material bedeckt sind, und in Rohrlängsrichtung gesehen bedeutend geringere Länge haben als die dazwischen liegenden lichtundurchlässigen Abschnitte.

2. Anordnung nach Anspruch 1, dadurch gekennzeichnet, dass mechanische Abschirmorgane in Form von rotierbaren, mit Schlitzen oder ähnlichen Öffnungen und ansonsten lichtundurchlässigen Zylindern um die Lichtquellen herum angeordnet sind, um die Länge der Zeit zu regeln, während welcher die Lichtquellen Licht auf die lichtdurchlässigen Abschnitte der Rohre in den Rohrstapeln werfen.

3. Anordnung nach Anspruch 1, dadurch gekennzeichnet, dass elektronische Steuerorgane an die Lichtquellen angeschlossen und dazu vorgesehen sind, dieses Licht während kürzeren, von dunklen Perioden unterbrochenen Perioden abzugeben.

4. Anordnung nach Anspruch 1, dadurch gekennzeichnet, dass das durch wenigstens einige der lichtdurchlässigen Abschnitte hindurch in die strömende Algendispersion eindringende Licht verschiedene Farben hat, zweckmässiger weise weiss, rot und blau.

5. Anordnung nach Anspruch 1, dadurch gekennzeichnet, dass das Verhältnis zwischen der Zeit, während welcher die Algen in der Flüssigkeit im Dunklen gehalten werden, zu der Zeit, während welcher sie beleuchtet sind, 10 : 1 beträgt.

**Revendications**

1. Installation de culture d'algues, de préférence d'algues microscopiques, comprenant une cuve dans laquelle les algues sont amenées à croître dans de l'eau en circulation et qui consiste en un système en canal fermé, ou un système analogue, réalisé sous la forme d'un ou plusieurs tuyaux en serpentins hélicoïdaux disposés de manière approprié en des piles communiquantes associées par paires (2a, 2b) et dotées de parties (9) de transmission de lumière, et une ou plusieurs sources de lumière, de préférence sous la forme de lampes fluorescentes (11), disposées au niveau des parties (9) de transmission de lumière, caractérisée en ce qu'au moins deux tuyaux en serpentins hélicoïdaux, ou piles, (2a, 2b) dans lesquels les tuyaux (1) dotés de parties de transmission de lumière et formant les serpentins, ou piles, et comportant de préférence une section droite rectangulaire, sont disposés au voisinage l'un de l'autre, font partie du système en canal prévu dans un espace tempéré et comprenant également un moyen servant à fournir une dispersion d'algues servant de solution de base, d'une solution nutritive, du dioxyde de carbone, de l'air, etc., et un espace d'expansion suffisant ainsi que des moyens servant à retirer l'eau avec les algues complètement développées, en ce que les serpentins, ou piles, (2a, 2b) sont reliés entre eux de façon que, dans une paire de ces serpentins, ou piles, l'écoulement se produise vers le haut pour l'un d'eux et vers le bas pour l'autre desdits serpentins, ou piles, et en ce que les parties (9) de transmission de lumière consistent en des ouvertures de diffusion ménagées dans la paroi du tuyau, laquelle est par ailleurs imperméable à la lumière, sont recouvertes de plaques, ou parties (10) de verre ou d'un matériau translucide analogue et, lorsqu'on les observe suivant la direction longitudinale du tuyau, présentent une longueur notablement plus petite que les parties intermédiaires imperméables à la lumière.

2. Installation selon la revendication 1, caractérisée en ce que des moyens faisant effet d'écrans mécaniques, qui se présentent sous la forme de cylindres rotatifs dotés de fentes ou d'ouvertures analogues, mais, pour le reste, imperméables à la lumière, sont disposés autour des sources lumineuses de manière à permettre un ajustement de la durée pendant laquelle les sources lumineuses projettent de la lumière en direction et au travers des parties (9) de transmission de lumière des tuyaux appartenant aux piles de tuyaux.

3. Installation selon la revendication 1, caractérisée en ce que des moyens de commande électroniques sont connectés aux sources lumineuses et sont destinés à faire que celles-ci émettent de la lumière pendant de brèves durées interrompues par des durées d'obscurité.

4. Installation selon la revendication 1, caractérisée en ce que la lumière pénétrant par au moins certaines des parties de transmission de lumière et dans la dispersion d'algues en circulation possède des couleurs différentes, lesquelles peuvent être de manière appropriée le blanc, le rouge et le bleu.

5. Installation selon la revendication 1, caractérisée en ce que le rapport de la durée pendant laquelle les algues contenues dans le liquide sont maintenues dans l'obscurité à la durée pendant laquelle elles sont illuminées est de 10 : 1.

FIG.1

FIG.2

FIG. 3

(3)

FIG. 4

EP 0 214 271 B1